# EUROPEAN PATENT APPLICATION

(11) **EP 4 603 122 A1**
(43) Date of publication of application: **20.08.2025**
(21) Application number: 22962136.2
(22) Date of filing: 27.12.2022
(51) Int. Cl.: A61M 5/32

(54) **SYRINGE NEEDLE COVER AND SYRINGE NEEDLE ASSEMBLY**

(30) Priority: 13.10.2022 JP 2022164478
(71) Applicant: UNISIS Corporation, Tokyo 110-0016 (JP)
(72) Inventor: SAITO, Hideya, Tokyo 110-0016 (JP); SARUYA, Akihiro, Koshigaya City, Saitama 3430822 (JP); MATSUMOTO, Yoshikazu, Koshigaya City, Saitama 3430822 (JP); YAMAGUCHI, Shohei, Tokyo 161-0033 (JP)
(74) Representative: Haseltine Lake Kempner LLP
(86) International application number: PCT/JP2022/048139
(87) International publication number: WO 2024/079919

(57) **Abstract**

Provided is an injection needle cover capable of improving operability while maintaining safety by protecting a needle shaft during transportation and preventing accidental puncture.

An injection needle cover 20 includes a support member 30 supporting a needle body including a needle shaft, a first member 50 rotatably connected to the support member 30 via a hinge 40 and covering a needle tip of the needle shaft, and a second member 60 separably connected to the first member 50 and constituting, with the first member, a container 70 accommodating the needle shaft. The injection needle cover is configured to be in one of: a first state in which the first member 50 and the second member 60 are connected to each other to accommodate the needle shaft in the container 70; a second state in which the second member 60 is separated from the first member 50 and the first member 50 is rotated from its position in the first state to expose the needle tip; and a third state in which the first member 50 is rotated from its position in the second state to cover the needle tip with the first member 50.

## Description

### TECHNICAL FIELD

The present invention relates to an injection needle cover and an injection needle assembly.

### BACKGROUND ART

There is known an injection needle assembly having an accidental puncture prevention function for preventing a user, such as a medical worker, from accidentally puncturing their body with a needle used for a patient or the like. The needle tip of the injection needle assembly is covered with an injection needle cover after puncture to prevent accidental puncture.

For example, PTL 1 describes a safety needle assembly including a cannula, a protector that covers the cannula and is detachable, and a sheath including an opening capable of accommodating the cannula. The safety needle assembly described in PTL 1 can prevent accidental puncture by covering the cannula with the sheath after puncture.

### CITATION LIST

### Patent Literature

PTL 1: WO 2012/111560 A1

### SUMMARY OF INVENTION

### Technical Problem

However, in the safety needle assembly described in PTL 1, since the sheath having an accidental puncture prevention function and the protector of the cannula are configured as independent mechanisms, the sheath and the protector cannot be compactly formed and are bulky, which makes removal of the protector and rotation of the sheath bothersome, and the bulkiness of the sheath can obstruct the field of view during injection operations requiring precision, which makes it cumbersome.

Furthermore, in the safety needle assembly described above, it is desirable to consider the environmental impact and reduce resource consumption.

### Solution to Problem

An injection needle cover according to an aspect of the invention includes:
a support member supporting a needle body including a needle shaft;
a first member rotatably connected to the support member via a hinge and covering a needle tip of the needle shaft; and
a second member separably connected to the first member and constituting, with the first member, a container accommodating the needle shaft, wherein
the injection needle cover is configured to be in one of:
   a first state in which the first member and the second member are connected to each other to accommodate the needle shaft in the container;
   a second state in which the second member is separated from the first member and the first member is rotated from its position in the first state to expose the needle tip; and
   a third state in which the first member is rotated from its position in the second state to cover the needle tip with the first member.

In the above aspect of the injection needle cover,
the first member may include a second fitting part fitted to a first fitting part of the support member in the second state.

In the above aspect of the injection needle cover,
the second member may include:
a protrusion to be pressed by a finger of a user; and
a flip-up part to flip up the first member when the protrusion is pressed by the finger of the user and the second member is separated from the first member, wherein
the first member is flipped up and rotated by the flip-up part.

In the above aspect of the injection needle cover,
the protrusion may include a pressed surface to be pressed by the finger of the user, and
the pressed surface may have a largest area among surfaces of the protrusion.

In the above aspect of the injection needle cover,
the second member may include a second engaging part to be engaged with a first engaging part of the support member in the first state, and
the first engaging part and the second engaging part may lock an inclination of the second member toward the needle shaft in the first state.

In the above aspect of the injection needle cover,
the support member, the hinge, and the first member may be integrally provided.

The above aspect of the injection needle cover may include:
a lock to maintain the connection between the first member and the second member in the first state.

In the above aspect of the injection needle cover,
the second member may be rotatably connected to the support member via the hinge, and
the needle shaft may be accommodated in the container in the third state.

In the above aspect of the injection needle cover,
the second member may include a grip part for gripping the second member.

In the above aspect of the injection needle cover,
the first member and the second member may be transparent and have different colors from each other.

An injection needle assembly according to an aspect of the invention includes:
the above aspect of the injection needle cover; and
the needle body.

In the above aspect of the injection needle assembly,
the needle body may include a needle hub supporting the needle shaft, and
the needle hub may be provided integrally with the support member.

The above aspect of the injection needle assembly may include:
a syringe connected to the needle body, wherein
the needle body includes a needle hub supporting the needle shaft, and
the needle hub is embedded in the syringe.

### BRIEF DESCRIPTION OF DRAWINGS

Fig. 1 is a perspective view schematically illustrating an injection needle assembly according to the first embodiment.
Fig. 2 is a perspective view schematically illustrating the injection needle assembly according to the first embodiment.
Fig. 3 is a side view schematically illustrating the injection needle assembly according to the first embodiment.
Fig. 4 is a side view schematically illustrating the injection needle assembly according to the first embodiment.
Fig. 5 is a side view schematically illustrating the injection needle assembly according to the first embodiment.
Fig. 6 is a side view schematically illustrating the injection needle assembly according to the first embodiment.
Fig. 7 is a side view schematically illustrating the injection needle assembly according to the first embodiment.
Fig. 8 is a side view schematically illustrating the injection needle assembly according to the first embodiment.
Fig. 9 is a side view schematically illustrating an injection needle assembly according to the second embodiment.
Fig. 10 is a side view schematically illustrating the injection needle assembly according to the second embodiment.
Fig. 11 is a side view schematically illustrating an injection needle assembly according to the third embodiment.
Fig. 12 is a side view schematically illustrating the injection needle assembly according to the third embodiment.
Fig. 13 is a side view schematically illustrating the injection needle assembly according to the third embodiment.
Fig. 14 is a side view schematically illustrating an injection needle assembly according to the fourth embodiment.
Fig. 15 is a side view schematically illustrating the injection needle assembly according to the fourth embodiment.
Fig. 16 is a side view schematically illustrating the injection needle assembly according to the fourth embodiment.
Fig. 17 is a side view schematically illustrating the injection needle assembly according to the fourth embodiment.
Fig. 18 is a side view schematically illustrating an injection needle assembly according to the fifth embodiment.
Fig. 19 is a side view schematically illustrating the injection needle assembly according to the fifth embodiment.
Fig. 20 is a side view schematically illustrating the injection needle assembly according to the fifth embodiment.
Fig. 21 is a side view schematically illustrating the injection needle assembly according to the fifth embodiment.
Fig. 22 is a side view schematically illustrating an injection needle assembly according to the sixth embodiment.

### DESCRIPTION OF EMBODIMENTS

Preferred embodiments of the invention will be described in detail with reference to the drawings. Note that the embodiments described below do not unduly limit the contents of the invention described in the claims. In addition, not all the components described below are essential components of the invention.

### 1. First embodiment

### 1.1. Configuration

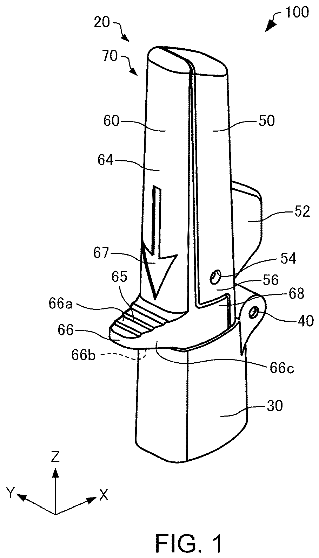

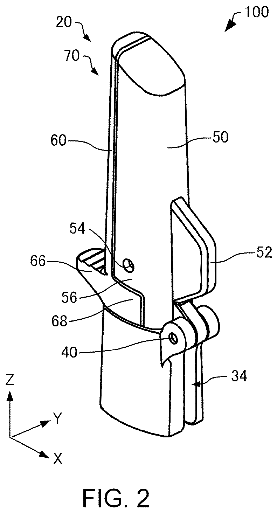

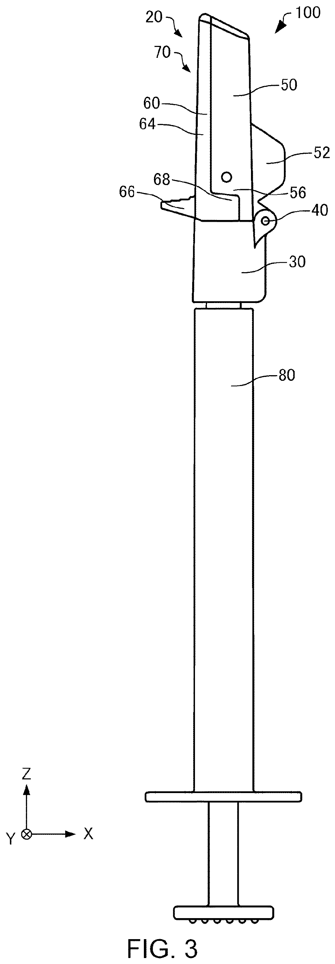

First, an injection needle assembly according to the first embodiment will be described with reference to the drawings. Figs. 1 and 2 are perspective views schematically illustrating an injection needle assembly 100 according to the first embodiment. Figs. 3 to 8 are side views schematically illustrating the injection needle assembly 100 according to the first embodiment. Note that, in Figs. 1 to 8, an X axis, a Y axis, and a Z axis are illustrated as three axes orthogonal to each other.

As illustrated in Figs. 1 to 8, the injection needle assembly 100 includes, for example, a needle body 10, an injection needle cover 20, and a syringe 80. The injection needle assembly 100 is used for intramuscular injection, hypodermic injection, and the like, for example.

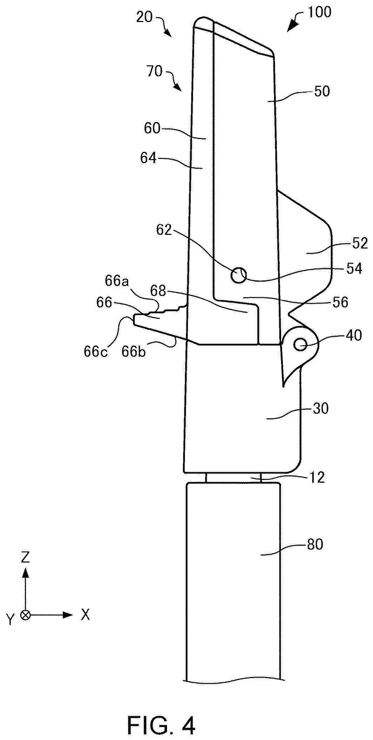

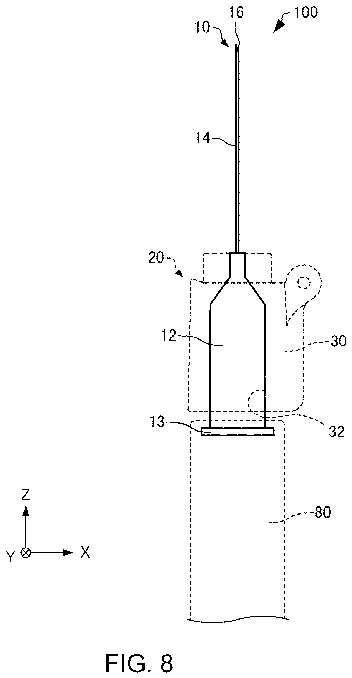

Note that, for convenience, the syringe 80 is not illustrated in Figs. 1 and 2. In addition, Fig. 3 illustrates the entire injection needle assembly 100, and Fig. 4 is an enlarged view of Fig. 3. In Fig. 8, the injection needle cover 20 and the syringe 80 are illustrated in transparent form. In Fig. 8, a first member 50 and a second member 60 of the injection needle cover 20 are not illustrated.

As illustrated in Fig. 8, the needle body 10 is supported by the injection needle cover 20. The needle body 10 includes, for example, a needle hub 12 and a needle shaft 14.

The needle hub 12 supports the needle shaft 14. The needle hub 12 includes a screwing part 13 to be screwed with the syringe 80. The screwing part 13 is exposed from the injection needle cover 20. In the illustrated example, the screwing part 13 is provided at an end of the needle hub 12 in the -Z axis direction.

The needle shaft 14 is connected to the needle hub 12. In the illustrated example, the needle shaft 14 extends from the needle hub 12 in the +Z axis direction. The needle shaft 14 includes a needle tip 16 to be inserted into a patient or the like when the injection needle assembly 100 is used. In the illustrated example, the needle tip 16 is a tip of the needle shaft 14 in the +Z axis direction.

As illustrated in Figs. 1 to 8, the injection needle cover 20 includes, for example, a support member 30, a hinge 40, a first member 50, and a second member 60. The injection needle cover 20 prevents accidental puncture of the needle body 10.

As illustrated in Fig. 8, the support member 30 supports the needle body 10. Specifically, the support member 30 supports the needle hub 12. The support member 30 is formed with an opening 32. The needle hub 12 is inserted in the opening 32 and fixed to the support member 30. The needle hub 12 and the support member 30 are integrally provided. The needle hub 12 and the support member 30 are integrally formed by, for example, injection molding, a 3D printer, or the like. The material of the needle hub 12 and the support member 30 is, for example, a resin, such as polypropylene (PP), polycarbonate (PC), or acrylonitrile butadiene styrene (ABS) resin. Note that the needle hub 12 and the support member 30 may be provided as separate parts.

As illustrated in Figs. 1 to 7, the hinge 40 connects the support member 30 and the first member 50. In the illustrated example, the hinge 40 is constituted by a pin extending in a direction orthogonal to the Z axis. The hinge 40 rotatably connects the first member 50 to the support member 30.

The first member 50 is rotatably connected to the support member 30 via the hinge 40. The first member 50 is rotatable within a range of 180° around the hinge 40, for example. The first member 50 covers the needle tip 16 of the needle shaft 14. The first member 50 has, for example, a shape that accommodates the entire needle shaft 14. The material of the first member 50 is, for example, the same as that of the support member 30. The first member 50 is manufactured, for example, in the same manner as the support member 30.

As illustrated in Fig. 2, the first member 50 includes a second fitting part 52 that can be fitted to a first fitting part 34 of the support member 30. The support member 30 includes the first fitting part 34 constituted by a notch formed in the support member 30, for example. The first member 50 includes the second fitting part 52 constituted by a protrusion, for example. Note that the first fitting part 34 may be a groove formed in the support member 30. Alternatively, the first fitting part 34 may be constituted by a groove, and the second fitting part 52 may be constituted by a protrusion.

The second member 60 is separably connected to the first member 50. As illustrated in Figs. 1 to 4, the second member 60 constitutes, with the first member, a container 70 that accommodates the needle shaft 14. The first member 50 and the second member 60 sandwich the needle shaft 14 from a direction orthogonal to the extending direction of the needle shaft 14 to constitute the container 70. The lengths of the first member 50 and the second member 60 in the Z axis direction are larger than the length of the needle shaft 14 in the Z axis direction. In the illustrated example, the container 70 accommodates the entire needle shaft 14.

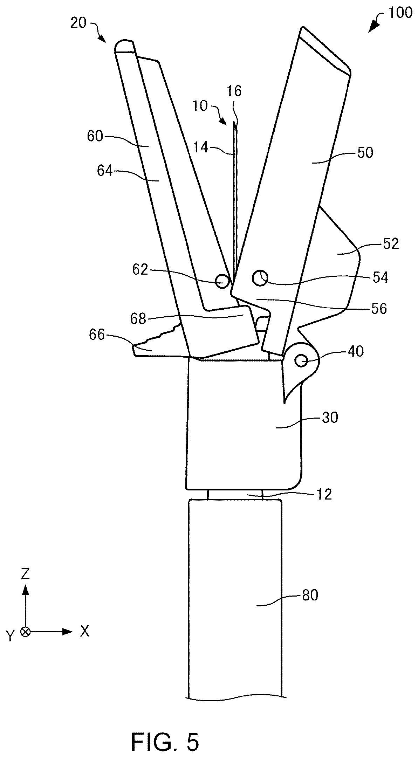

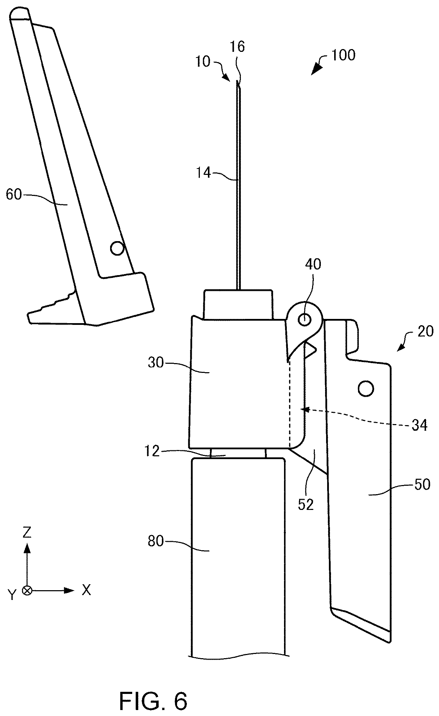

As illustrated in Figs. 5 and 6, the second member 60 is separated from the first member 50. The second member 60 includes a fitting part 62 to be fitted with a fitting part 54 of the first member 50. The first member 50 includes the fitting part 54 constituted by a through hole, for example. The second member 60 includes the fitting part 62 constituted by a protrusion. When the fitting between the fitting part 54 and the fitting part 62 is released, the second member 60 is separated from the first member 50. Note that the fitting part 54 may be constituted by a through hole, and the fitting part 62 may be constituted by a protrusion.

The material of the second member 60 is, for example, the same as that of the first member 50. The second member 60 is manufactured, for example, in the same manner as the first member 50. The first member 50 and the second member 60 are, for example, transparent. The first member 50 and the second member 60 have, for example, different colors from each other. The first member 50 is, for example, colorless and transparent. The second member 60 is, for example, colored and transparent. When the first member 50 and the second member 60 are transparent, the first member 50 and the second member 60 are, for example, PP or PC.

Note that as long as the first member 50 and the second member 60 are transparent and have different colors from each other, the second member 60 may be colorless and transparent, and the first member 50 may be colored and transparent. In addition, both the first member 50 and the second member 60 may be colored and transparent, such that the first member 50 is blue and transparent and the second member 60 is green and transparent.

The second member 60 includes, for example, a base part 64, a protrusion 66, and a flip-up part 68.

The base part 64 is configured to be connectable to the first member 50. In the example illustrated in Fig. 4, the length of the base part 64 in the Z axis direction is equal to or longer than the length of the first member 50 in the Z axis direction.

The protrusion 66 protrudes from the base part 64. The protrusion 66 is pressed by a finger of a user of the injection needle assembly 100. As a result, the second member 60 is separated from the first member 50 as illustrated in Fig. 6. The protrusion 66 is pressed, for example, in the -Z axis direction. As illustrated in Fig. 1, a mark 67 indicating a pressing direction is formed on the base part 64, for example. In the illustrated example, the mark 67 is an arrow pointing downward (in the -Z axis direction). The mark 67 is formed in the +Z axis direction of protrusion 66. In the illustrated example, the mark 67 is a recess formed in the base part 64. Note that the mark 67 may be, for example, a sticker or ink attached to the base part 64.

The protrusion 66 includes a pressed surface 66a to be pressed by a finger of a user, a bottom surface 66b facing the opposite side of the pressed surface 66a, and a side surface 66c connecting the pressed surface 66a and the bottom surface 66b. The pressed surface 66a has the largest area in the surface of the protrusion 66. The area of the pressed surface 66a may be larger than the area of the bottom surface 66b, or may be the same as the area of the bottom surface 66b. The area of the pressed surface 66a is larger than the area of the side surface 66c.

As illustrated in Fig. 1, the pressed surface 66a of the protrusion 66 is provided with a non-slip part 65 for preventing, for example, the finger of the user from slipping. The non-slip part 65 includes, for example, a plurality of steps extending in a direction orthogonal to the extending direction of the needle shaft 14. The plurality of steps is arranged, for example, in a direction orthogonal to the extending direction of the steps. Although not illustrated, the non-slip part 65 may be constituted by a plurality of small protrusions.

The flip-up part 68 protrudes from the base part 64 in a direction opposite to the protrusion 66. In the example in Fig. 4, the flip-up part 68 is in contact with the support member 30. The flip-up part 68 flips up the first member 50 when the protrusion 66 is pressed by the finger of the user and the second member 60 is separated from the first member 50. The first member 50 is flipped up and rotated by the flip-up part 68. In the illustrated example, the first member 50 includes a receiving part 56 in contact with the flip-up part 68. When the protrusion 66 is pressed, the flip-up part 68 moves while being in contact with the receiving part 56 as illustrated in Fig. 5, and flips up the first member 50 in a direction in which the first member 50 rotates.

The syringe 80 is connected to the needle hub 12 of the needle body 10. As illustrated in Fig. 8, the syringe 80 is screwed to the screwing part 13 of the needle hub 12, for example. The material of the syringe 80 is, for example, resin. When the injection needle assembly 100 is used, the liquid medicine contained in the syringe 80 is injected into a patient or the like through the needle body 10.

### 1.2. Movement

### 1.2.1. First state

The injection needle cover 20 of the injection needle assembly 100 is configured to be in a first state, a second state, and a third state.

In the first state, as illustrated in Figs. 1 to 4, the first member 50 and the second member 60 are connected to each other to accommodate the needle shaft 14 in the container 70. The needle tip 16 of the needle shaft 14 is covered with the first member 50. The needle tip 16 overlaps the first member 50 as viewed in the Z axis direction.

### 1.2.2. Second state

In the second state, as illustrated in Fig. 6, the second member 60 is separated from the first member 50, and the first member 50 is rotated from its position in the first state to expose the needle tip 16.

Specifically, the user presses the protrusion 66 of the second member 60 in the first state in the -Z axis direction with a finger. As a result, the second member 60 is separated from the first member 50. At the time of separation from the first member 50, as illustrated in Fig. 5, the flip-up part 68 of the second member 60 moves while being in contact with the receiving part 56 of the first member 50, and flips up and rotates the first member 50. The flip-up part 68 enables the user to rotate the first member 50 without touching the first member 50. In the illustrated example, the first member 50 is rotated clockwise.

Next, the user presses the first member 50 toward the support member 30. As a result, as illustrated in Fig. 6, the second fitting part 52 of the first member 50 is fitted to the first fitting part 34 of the support member 30.

As a result of the above, the injection needle cover 20 is in the second state in which the needle tip 16 is exposed. In the second state, the second member 60 is separated from the first member 50 and isolated from the support member 30. The second member 60 separated from the first member 50 is discarded, for example. The user punctures a patient or the like using the injection needle assembly 100 with the injection needle cover 20 in the second state.

### 1.2.3. Third state

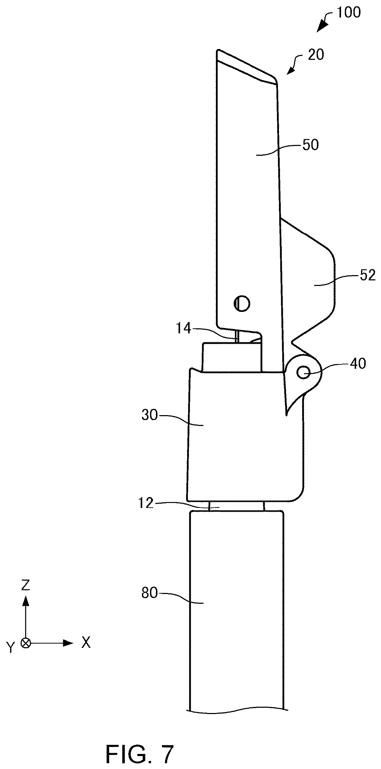

In the third state, as illustrated in Fig. 7, the first member 50 is rotated from its position in the second state, and the needle tip 16 is covered with the first member 50.

Specifically, after the puncture using the injection needle assembly 100 in the second state, the user releases the fitting between the first fitting part 34 of the support member 30 and the second fitting part 52 of the first member 50. Next, the user rotates the first member 50 to cover the needle tip 16 with the first member 50 as illustrated in Fig. 7. As a result, the injection needle cover 20 is in the third state. In the illustrated example, the first member 50 is rotated counterclockwise.

The injection needle cover 20 may have a configuration that prevents the first member 50 from rotating again in the third state. In other words, the injection needle cover 20 may have a fixing structure that prevents the first member 50 from rotating again in the third state. Although not illustrated, the first member 50 may be provided with a return prevention part to be fixed to the needle shaft 14, for example. As a result, the possibility of accidental puncture can be further reduced.

### 1.3. Effects

The injection needle cover 20 includes the support member 30 supporting the needle body 10 including the needle shaft 14, the first member 50 rotatably connected to the support member 30 via the hinge 40 and covering the needle tip 16 of the needle shaft 14, and the second member 60 separably connected to the first member 50 and constituting, with the first member, the container 70 accommodating the needle shaft 14. The injection needle cover 20 is configured to be in the first state in which the first member 50 and the second member 60 are connected to each other to accommodate the needle shaft 14 in the container 70, the second state in which the second member 60 is separated from the first member 50 and the first member 50 is rotated from its position in the first state to expose the needle tip 16, and the third state in which the first member 50 is rotated from its position in the second state to cover the needle tip 16 with the first member 50.

As described above, in the injection needle cover 20, the first member 50 covering the needle tip 16 in the third state constitutes the container 70 accommodating the needle shaft 14 in the first state. Therefore, as compared to a case in which a member covering the needle tip in the third state and a container accommodating the needle shaft in the first state are constituted by separate members, the injection needle cover 20 can improve the operability while maintaining the safety by protecting the needle shaft 14 during transportation and preventing accidental puncture. Furthermore, downsizing can be achieved, and resource consumption can be reduced.

In the injection needle cover 20, the first member 50 includes the second fitting part 52 fitted to the first fitting part 34 of the support member 30 in the second state. Therefore, the injection needle cover 20 can prevent the first member 50 from interfering with puncture by the injection needle assembly 100.

In the injection needle cover 20, the second member 60 includes the protrusion 66 to be pressed by a finger of a user and the flip-up part 68 that flips up the first member 50 when the protrusion 66 is pressed by the finger of the user and the second member 60 is separated from the first member 50, and the first member 50 is flipped up and rotated by the flip-up part 68. Therefore, the user of the injection needle assembly 100 can change the injection needle cover 20 from the first state to the second state only with one finger (for example, the thumb) of the hand gripping the syringe 80, for example. In this manner, the user can easily change the injection needle assembly 100 from the first state to the second state by a single action with one hand and one finger (by pressing the protrusion 66 with the thumb). As a result, the labor of the user can be reduced.

Furthermore, since the injection needle cover 20 can be changed from the first state to the second state with one hand gripping the syringe 80, there is less possibility of accidentally puncturing the other hand with the injection needle assembly 100. For example, in a case in which the syringe is gripped with one hand and the container is removed from the support member with the other hand to expose the needle shaft, the other hand can be accidentally punctured due to excessive force used to remove the container.

In the injection needle cover 20, the protrusion 66 includes the pressed surface 66a to be pressed by a finger of the user, and the pressed surface 66a has the largest area in the surface of the protrusion 66. Therefore, the user can easily press the pressed surface 66a.

In the injection needle cover 20, the first member 50 and the second member 60 are transparent and have different colors from each other. Therefore, the user can visually recognize the needle shaft 14 while the needle shaft 14 is accommodated in the container 70. Furthermore, the user can recognize that the container 70 is constituted by two parts.

The injection needle assembly 100 includes the injection needle cover 20 and the needle body 10. Therefore, resource consumption can be reduced with the injection needle assembly 100.

In the injection needle assembly 100, the needle body 10 includes the needle hub 12 supporting the needle shaft 14, and the needle hub 12 is provided integrally with the support member 30. Therefore, the number of parts of the injection needle assembly 100 can be reduced. Accordingly, cost reduction can be achieved.

### 2. Second embodiment

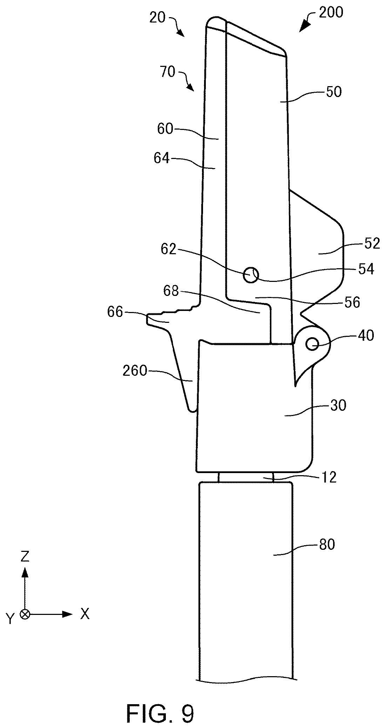

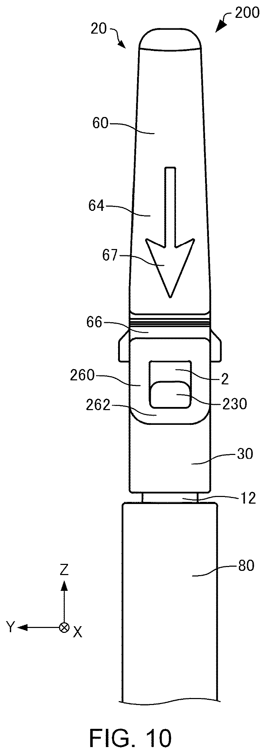

Next, an injection needle assembly according to the second embodiment will be described with reference to the drawings. Figs. 9 and 10 are side views schematically illustrating an injection needle assembly 200 according to the second embodiment. Note that Figs. 9 and 10 are views from directions orthogonal to each other. Figs. 9 and 10 illustrate the first state.

Hereinafter, in the injection needle assembly 200 according to the second embodiment, members having the same functions as the component members of the injection needle assembly 100 according to the first embodiment described above are denoted by the same reference signs, and the detailed description thereof will be omitted. The same applies to injection needle assemblies according to the third to sixth embodiments, which will be described later.

As illustrated in Figs. 9 and 10, the injection needle cover 20 of the injection needle assembly 200 is different from the injection needle cover 20 of the injection needle assembly 100 described above in that the support member 30 includes a first engaging part 230 and the second member 60 includes a second engaging part 260.

The second engaging part 260 of the second member 60 is engaged with the first engaging part 230 of the support member 30 in the first state. The first engaging part 230 is, for example, a protrusion. The second engaging part 260 has a shape that is caught by the first engaging part 230 in the first state. As illustrated in Fig. 10, the second engaging part 260 includes, for example, a rod-shaped part 262 in contact with the first engaging part 230 in the first state. In the illustrated example, the second engaging part 260 is connected to the protrusion 66.

The first engaging part 230 and the second engaging part 260 lock the inclination of the second member 60 toward the needle shaft 14 in the first state. In other words, the second member 60 is not inclined toward the needle shaft 14 in the first state due to the engagement between the first engaging part 230 and the second engaging part 260. In the illustrated example, the second member 60 is not inclined in the +X axis direction in the first state.

In the first state, on the opposite side of the first engaging part 230 to the rod-shaped part 262 (in the illustrated example, in the +Z axis direction of the first engaging part 230), a gap 2 is provided. As a result, the second member 60 is not inclined toward the needle shaft 14, but is rotatable to the side opposite to the needle shaft 14.

Note that the shapes of the first engaging part 230 and the second engaging part 260 are not particularly limited as long as the second member 60 is not inclined toward the needle shaft 14 in the first state due to the engagement between the first engaging part 230 and the second engaging part 260.

In the injection needle cover 20 of the injection needle assembly 200, the second member 60 includes the second engaging part 260 engaged with the first engaging part 230 of the support member 30 in the first state, and the first engaging part 230 and the second engaging part 260 lock the inclination of the second member 60 toward the needle shaft 14 in the first state. Therefore, the injection needle cover 20 can prevent the second member 60 from being brought into contact with the needle shaft 14 even if the second member 60 is pushed toward the needle shaft 14 in the first state. As a result, the needle shaft 14 can be prevented from being deformed by the second member 60.

### 3. Third embodiment

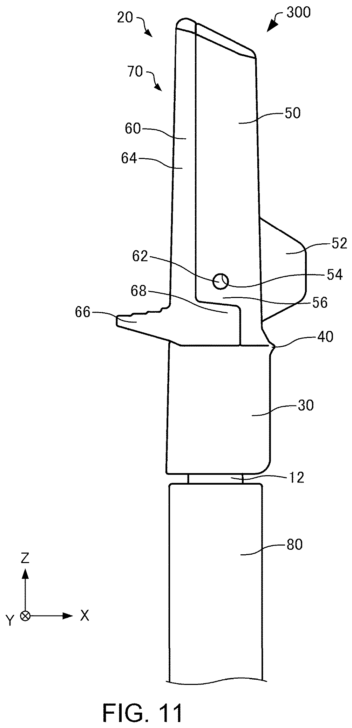

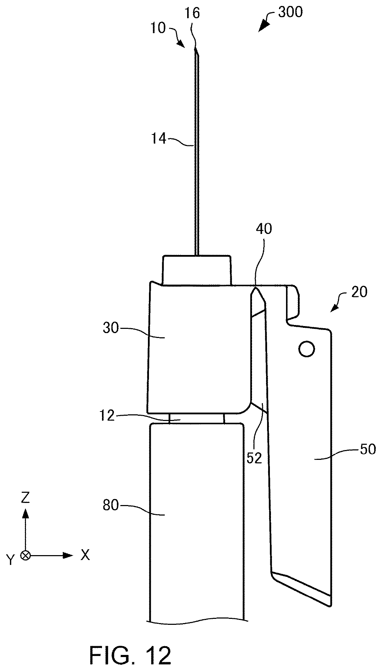

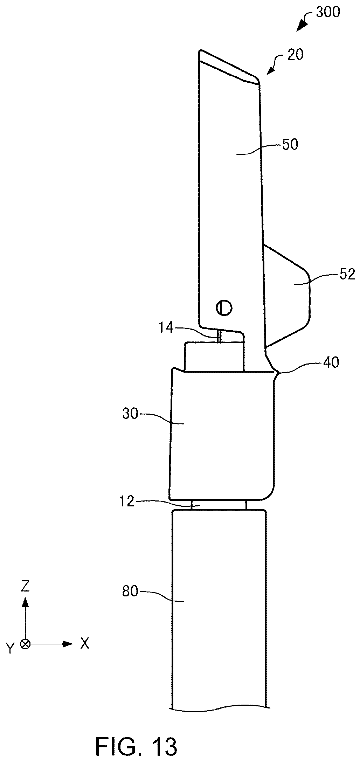

Next, an injection needle assembly according to the third embodiment will be described with reference to the drawings. Figs. 11 to 13 are side views schematically illustrating an injection needle assembly 300 according to the third embodiment. Fig. 11 illustrates the first state. Fig. 12 illustrates the second state. Fig. 13 illustrates the third state.

In the injection needle cover 20 of the injection needle assembly 100 described above, the hinge 40 is a pin, and the support member 30, the hinge 40, and the first member 50 are configured as separate members, as illustrated in Figs. 4 to 7.

In contrast to this, in the injection needle cover 20 of the injection needle assembly 300, the support member 30, the hinge 40, and the first member 50 are integrally provided, as illustrated in Figs. 11 to 13. The support member 30, the hinge 40, and the first member 50 are integrally formed by, for example, injection molding, a 3D printer, or the like. The hinge 40 is a connecting part between the support member 30 and the first member 50. The materials of the support member 30, the hinge 40, and the first member 50 are the same as each other.

In the injection needle cover 20 of the injection needle assembly 300, the support member 30, the hinge 40, and the first member 50 are integrally provided. Therefore, the injection needle cover 20 can reduce the number of parts. Accordingly, cost reduction can be achieved.

### 4. Fourth embodiment

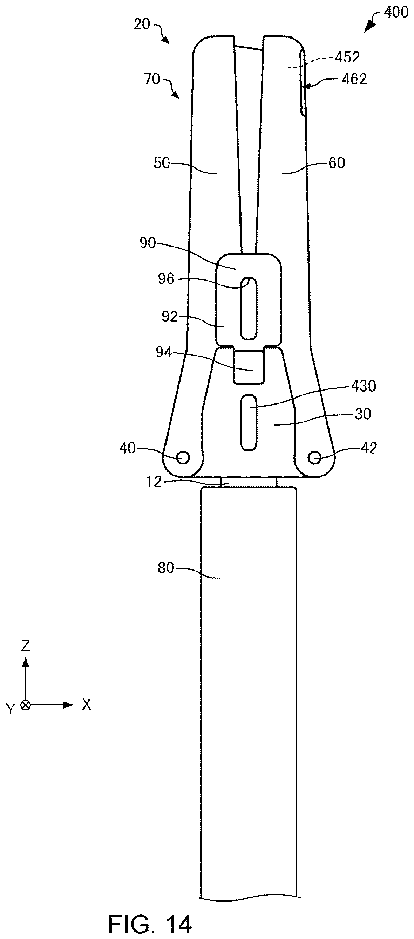

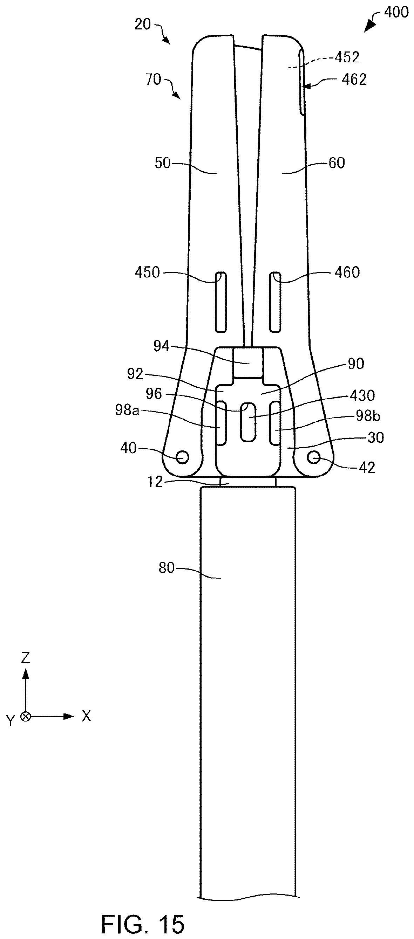

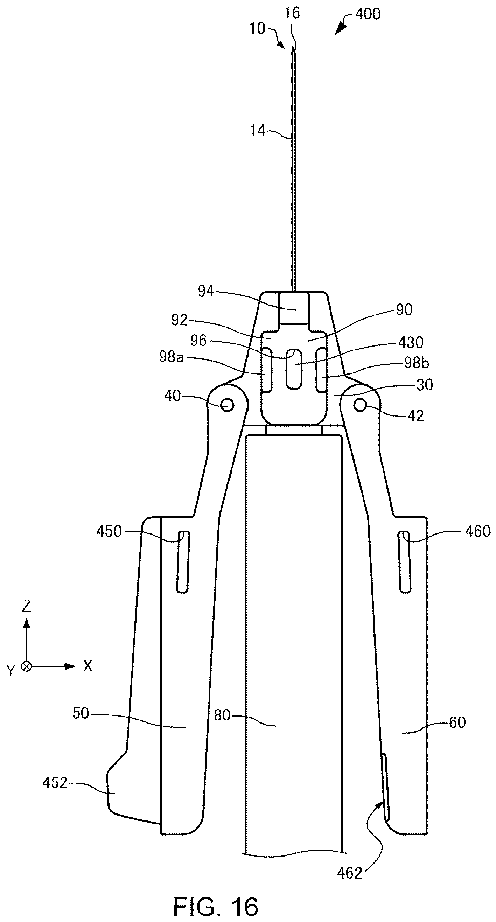

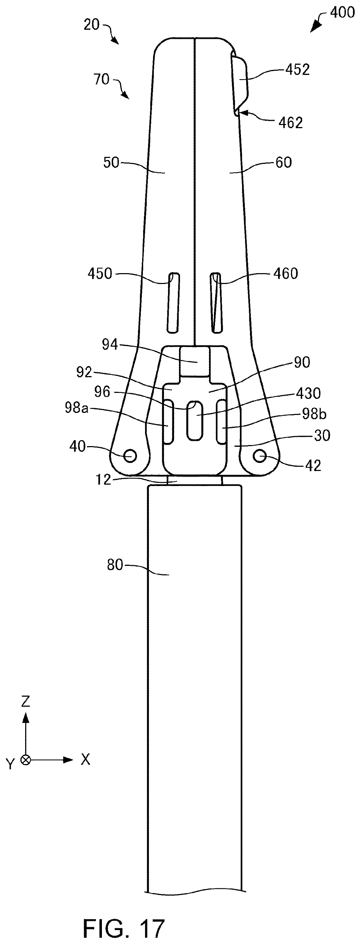

Next, an injection needle assembly according to the fourth embodiment will be described with reference to the drawings. Figs. 14 to 17 are side views schematically illustrating an injection needle assembly 400 according to the fourth embodiment. Figs. 14 and 15 illustrate the first state. Fig. 16 illustrates the second state. Fig. 17 illustrates the third state.

As illustrated in Figs. 14 to 17, the injection needle cover 20 of the injection needle assembly 400 is different from the injection needle cover 20 of the injection needle assembly 100 described above in that a lock 90 is included.

The lock 90 is connected to the support member 30. As illustrated in Figs. 14 and 15, the lock 90 includes a base part 92, a hinge 94, and fitting parts 96, 98a, and 98b.

The base part 92 is rotatably connected to the support member 30 via the hinge 94. The fitting part 96 is, for example, a through hole formed in the base part 92. The support member 30 includes, for example, a fitting part 430 constituted by a protrusion. In the example illustrated in Fig. 15, the fitting part 96 and the fitting part 430 are fitted. In the example illustrated in Fig. 14, the fitting between the fitting part 96 and the fitting part 430 is released.

As illustrated in Fig. 15, the fitting parts 98a and 98b are provided on the base part 92. The fitting parts 98a and 98b are, for example, protrusions provided on the base part 92. In the illustrated example, the fitting part 96 is formed between the fitting parts 98a and 98b. The first member 50 is provided with, for example, a fitting part 450 constituted by an opening. The fitting part 98a is to be fitted to the fitting part 450. The second member 60 is provided with, for example, a fitting part 460 constituted by an opening. The fitting part 98a is to be fitted to the fitting part 460.

When the fitting part 98a is fitted to the fitting part 450 and the fitting part 98b is fitted to the fitting part 460, the lock 90 supports the connection between the first member 50 and the second member 60 in the first state as illustrated in Fig. 14. While the fitting part 98a is fitted to the fitting part 450 and the fitting part 98b is fitted to the fitting part 460, the first member 50 and the second member 60 are not rotated.

The first member 50 further includes, for example, a fitting part 452 constituted by a protrusion. The second member 60 includes, for example, a fitting part 462 constituted by a through hole. The fitting part 452 is fitted to the fitting part 462.

The injection needle cover 20 includes a hinge 42. The second member 60 is rotatably connected to the support member 30 via the hinge 42. The extending direction of the hinge 42 is the same as the extending direction of the hinge 40.

When the injection needle cover 20 of the injection needle assembly 400 is changed from the first state to the second state, a user rotates the lock 90 to release the fitting between the fitting part 98a and the fitting part 450 and the fitting between the fitting part 98b and the fitting part 460 as illustrated in Fig. 15. Then, the fitting part 96 and the fitting part 430 are fitted to each other.

Next, the user rotates the first member 50 and the second member 60 in directions opposite to each other to separate them. As a result, the injection needle cover 20 is in the second state as illustrated in Fig. 16.

When changing from the second state to the third state, the user rotates the first member 50 in the direction opposite to the rotation from the first state to the second state. In addition, the second member 60 is rotated in the direction opposite to the rotation from the first state to the second state. Then, the first member 50 and the second member 60 are brought into contact with each other. As a result, the injection needle cover 20 is in the third state as illustrated in Fig. 17. In the third state, the injection needle cover 20 accommodates the needle shaft 14 in the container 70 constituted by the first member 50 and the second member 60.

Next, the user further pushes the first member 50 and the second member 60 in the direction opposite to the rotation from the first state to the second state while the first member 50 and the second member 60 are in contact with each other. As a result, the fitting part 452 of the first member 50 and the fitting part 462 of the second member 60 are fitted to each other and, for example, are in a fixed state in which re-rotation is prevented.

The injection needle cover 20 of the injection needle assembly 400 includes the lock 90 that maintains the connection between the first member 50 and the second member 60 in the first state. Therefore, the connection between the first member 50 and the second member 60 can be maintained, even if a force is applied to at least one of the first member 50 and the second member 60 in the first state to rotate them in a direction in which the connection between the first member 50 and the second member 60 is released. As a result, the possibility of accidental puncture can be further reduced.

In the injection needle cover 20 of the injection needle assembly 400, the second member 60 is rotatably connected to the support member 30 via the hinge 42, and the needle shaft 14 is accommodated in the container 70 in the third state. Therefore, in the injection needle cover 20 of the injection needle assembly 400, the exposure of the needle shaft 14 in the third state can be reduced as compared with, for example, the injection needle cover 20 of the injection needle assembly 100.

### 5. Fifth embodiment

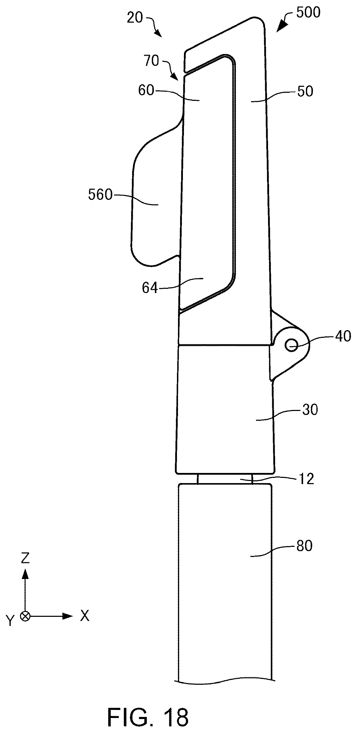

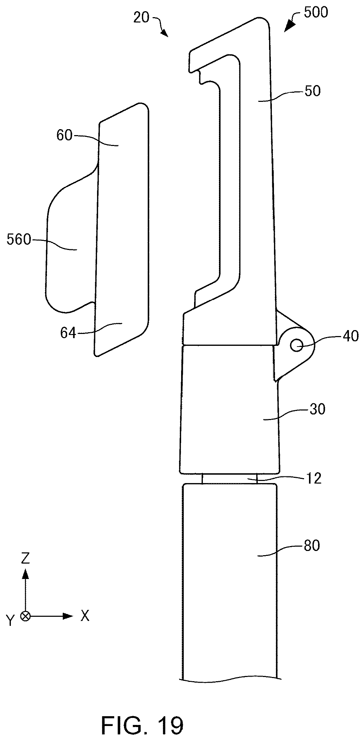

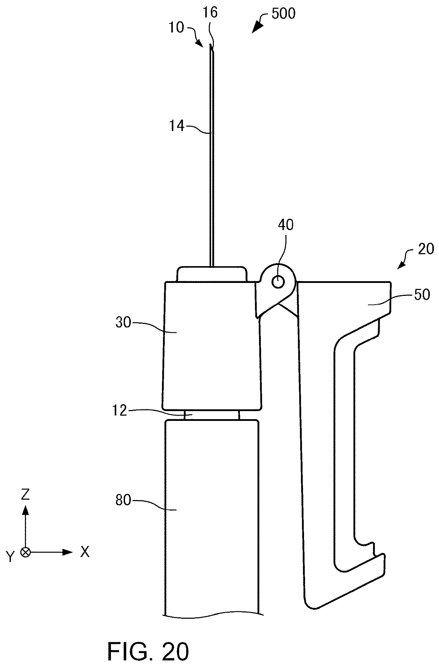

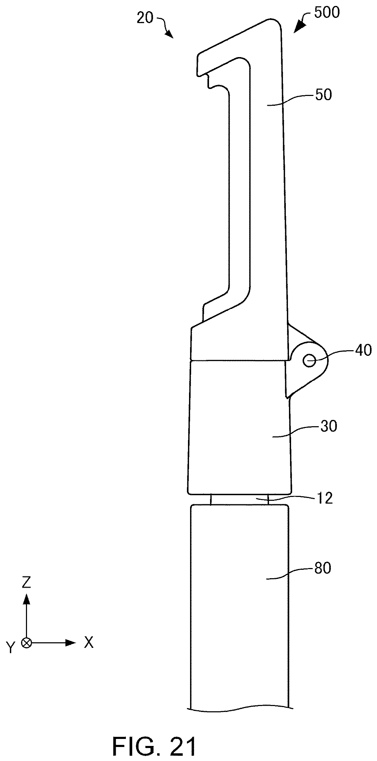

Next, an injection needle assembly according to the fifth embodiment will be described with reference to the drawings. Figs. 18 to 21 are side views schematically illustrating an injection needle assembly 500 according to the fifth embodiment. Fig. 18 illustrates the first state. Fig. 19 illustrates an intermediate state from the first state to the second state. Fig. 20 illustrates the second state. Fig. 21 illustrates the third state.

In the injection needle cover 20 of the injection needle assembly 100 described above, the second member 60 includes the protrusion 66 to be pressed by a finger of a user as illustrated in Figs. 4 and 5.

In contrast to this, in the injection needle cover 20 of the injection needle assembly 500, the second member 60 includes a grip part 560 for gripping the second member 60 as illustrated in Figs. 18 and 19. The grip part 560 has a shape in which the extending direction of the needle shaft 14 is the longitudinal direction in the first state.

In the injection needle cover 20 of the injection needle assembly 500, the user grips the grip part 560 with, for example, the thumb and the index finger when the first state is changed to the second state. Then, the user pulls the grip part 560 in, for example, a direction intersecting with the extending direction of the needle shaft 14 to separate the second member 60 from the first member 50 as illustrated in Fig. 19. Next, the user rotates the first member 50. As a result, the injection needle cover 20 is in the second state as illustrated in Fig. 20. Next, the user rotates the first member 50 in a direction opposite to the rotation from the first state to the second state. As a result, the injection needle cover 20 is in the third state as illustrated in Fig. 21.

In the injection needle cover 20 of the injection needle assembly 500, the second member 60 includes the grip part 560 for gripping the second member 60. Therefore, the user of the injection needle assembly 500 can grip the grip part 560 to separate the second member 60 from the first member 50.

### 6. Sixth embodiment

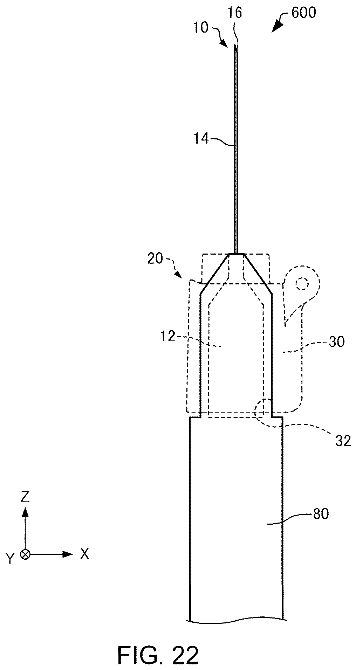

Next, an injection needle assembly according to the sixth embodiment will be described with reference to the drawings. Fig. 22 is a side view schematically illustrating an injection needle assembly 600 according to the sixth embodiment. Note that, for convenience, members other than the needle body 10, the support member 30, and the syringe 80 are not illustrated in Fig. 22. In addition, the needle hub 12 and the support member 30 are indicated by broken lines.

As illustrated in Fig. 8, in the injection needle assembly 100 described above, the needle hub 12 includes the screwing part 13 to be screwed with the syringe 80, and can be separated from the syringe 80.

In contrast to this, in the injection needle assembly 600, the needle hub 12 does not include the screwing part 13 to be screwed with the syringe 80 as illustrated in Fig. 22. The needle hub 12 is embedded in the syringe 80. The syringe 80 is provided integrally with the needle hub 12, for example. The support member 30 supports the needle hub 12 via the syringe 80.

In the injection needle assembly 600, the needle hub 12 is embedded in the syringe 80. Therefore, when the injection needle assembly 600 is used, the work of attaching the syringe 80 to the needle hub 12 can be omitted.

The above-described embodiments and modification examples are illustrative only, and the invention is not limited thereto. For example, the respective embodiments and respective modification examples can appropriately be combined.

The invention includes configurations that are substantially the same (for example, configurations having the same functions, methods and results, or configurations having the same objectives and effects) as the configurations described in the embodiments. The invention also includes configurations obtained by replacing non-essential elements of the configurations described in the embodiments. The invention also includes configurations exhibiting the same operations and effects as those of the configurations described in the embodiments, or configurations capable of achieving the same objectives as those of the configurations described in the embodiments. The invention further includes configurations obtained by adding known art to the configurations described in the embodiments.

### REFERENCE SIGNS LIST

- 2: Gap
- 10: Needle body
- 12: Needle hub
- 13: Screwing part
- 14: Needle shaft
- 16: Needle tip
- 20: Injection needle cover
- 30: Support member
- 32: Opening
- 34: First fitting part
- 40, 42: Hinge
- 50: First member
- 52: Second fitting part
- 54: Fitting part
- 56: Receiving part
- 60: Second member
- 62: Fitting part
- 64: Base part
- 65: Non-slip part
- 66: Protrusion
- 66a: Pressed surface
- 66b: Bottom surface
- 66c: Side surface
- 67: Mark
- 68: Flip-up part
- 70: Container
- 80: Syringe
- 90: Lock
- 92: Base part
- 94: Hinge
- 96, 98a, 98b: Fitting part
- 100, 200: Injection needle assembly
- 230: First engaging part
- 260: Second engaging part
- 262: Rod-shaped part
- 300, 400: Injection needle assembly
- 450, 452, 460, 462: Fitting part
- 500: Injection needle assembly
- 560: Grip part
- 600: Injection needle assembly

## Claims

1. An injection needle cover comprising:
a support member supporting a needle body including a needle shaft;
a first member rotatably connected to the support member via a hinge and covering a needle tip of the needle shaft; and
a second member separably connected to the first member and constituting, with the first member, a container accommodating the needle shaft, wherein
the injection needle cover is configured to be in one of:
a first state in which the first member and the second member are connected to each other to accommodate the needle shaft in the container;
a second state in which the second member is separated from the first member and the first member is rotated from its position in the first state to expose the needle tip; and
a third state in which the first member is rotated from its position in the second state to cover the needle tip with the first member.

2. The injection needle cover according to claim 1, wherein
the first member includes a second fitting part fitted to a first fitting part of the support member in the second state.

3. The injection needle cover according to claim 1, wherein
the second member includes:
a protrusion to be pressed by a finger of a user; and
a flip-up part to flip up the first member when the protrusion is pressed by the finger of the user and the second member is separated from the first member, wherein
the first member is flipped up and rotated by the flip-up part.

4. The injection needle cover according to claim 3, wherein
the protrusion includes a pressed surface to be pressed by the finger of the user, and
the pressed surface has a largest area among surfaces of the protrusion.

5. The injection needle cover according to claim 3, wherein
the second member includes a second engaging part to be engaged with a first engaging part of the support member in the first state, and
the first engaging part and the second engaging part lock an inclination of the second member toward the needle shaft in the first state.

6. The injection needle cover according to claim 1, wherein
the support member, the hinge, and the first member are integrally provided.

7. The injection needle cover according to claim 1, comprising:
a lock to maintain the connection between the first member and the second member in the first state.

8. The injection needle cover according to claim 7, wherein
the second member is rotatably connected to the support member via the hinge, and
the needle shaft is accommodated in the container in the third state.

9. The injection needle cover according to claim 1, wherein
the second member includes a grip part for gripping the second member.

10. The injection needle cover according to claim 1, wherein
the first member and the second member are transparent and have different colors from each other.

11. An injection needle assembly comprising:
the injection needle cover according to any one of claims 1 to 10; and
the needle body.

12. The injection needle assembly according to claim 11, wherein
the needle body includes a needle hub supporting the needle shaft, and
the needle hub is provided integrally with the support member.

13. The injection needle assembly according to claim 11, comprising:
a syringe connected to the needle body, wherein
the needle body includes a needle hub supporting the needle shaft, and
the needle hub is embedded in the syringe.
